(19) Europäisches Patentamt
European Patent Office
Office européen des brevets

(11) Publication number : **0 653 434 A1**

(12) **EUROPEAN PATENT APPLICATION**

(21) Application number : **94308306.3**

(22) Date of filing : **10.11.94**

(51) Int. Cl.[6] : **C07H 15/12**, C07H 15/04, C07H 15/18

(30) Priority : **12.11.93 JP 283147/93**

(43) Date of publication of application : **17.05.95 Bulletin 95/20**

(84) Designated Contracting States : **AT BE CH DE DK ES FR GB GR IE IT LI LU MC NL PT SE**

(71) Applicant : **TANABE SEIYAKU CO., LTD.**
**2-10, Dosho-machi 3-chome**
**Chuo-ku Osaka (JP)**

(72) Inventor : **Kurita, Hironori**
**2-9-27, Kita-machi**
**Warabi-shi, Saitama-ken (JP)**
Inventor : **Imanishi, Yasuhiro**
**1-27-16, Kitasenzoku**
**Ota-ku, Tokyo-to (JP)**
Inventor : **Ishida, Akihiko**
**10-16-1-415, Tokiwa**
**Urawa-shi, Saitama-ken (JP)**
Inventor : **Onta, Tokio**
**2-29-6, Kamiishihara**
**Chofu-shi, Tokyo-to (JP)**
Inventor : **Ohashi, Motoaki**
**4-9-2, Maehara-cho**
**Koganei-shi, Tokyo-to (JP)**

(74) Representative : **Baverstock, Michael George Douglas et al**
**BOULT, WADE & TENNANT**
**27 Furnival Street**
**London, EC4A 1PQ (GB)**

(54) **Glucosamine derivative, process for preparing the same and synthetic intermediate thereof.**

(57) There are disclosed a D-glucosamine derivative represented by the formula (I) :

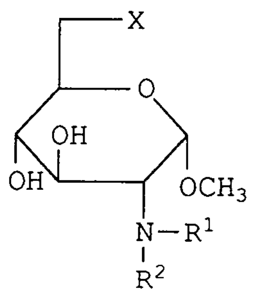

(I)

wherein (1) X is a group of formula :

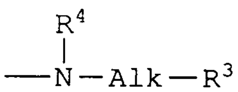

and $R^1$ and $R^2$ are both hydrogen atoms ; or (2) X is OH or a group of the formula :

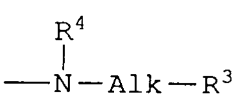

and one of $R^1$ and $R^2$ is a group of the formula : -Y-$R^{11}$ or alkyl and the other is H, or one of $R^1$ and $R^2$ is a group of the formula : -Y-$R^{11}$ and the other is alkyl ; Y is a group of the formula : -CO-, -CS- or -$SO_2$- ; $R^{11}$ is alkyl, lower alkoxy, alkenyl, alkynyl, phenyl, cycloalkyl, tricycloalkyl, heterocyclic or alkylamino ; $R^3$ is H, phenyl, phenoxy, phenylthio, phenylamino or heterocyclic ; $R^4$ is alkanoyl, alkenoyl or alkynoyl ; and Alk is alkylene, provided that a compound in which X is OH, one of $R^1$ and $R^2$ is methyl and the other is acetyl is excluded,
or a pharmaceutically acceptable salt thereof, a process for preparing the same, and a synthetic intermediate thereof.

EP 0 653 434 A1

BACKGROUND OF THE INVENTION

The present invention relates to a novel D-glucosamine derivative which has a leukocyte-increasing activity and an infection-preventing activity, processes for preparing the same and a synthetic intermediate thereof.

It has been known that leukocytes in a living body, particularly neutrophils, macrophages and lymphocytes have a preventing activity against bacterial or fungal infection, anti-tumor activity or an extensive immunological activity with a wide spectrum by enhancing functions thereof.

As a compound having such an activity, particularly an infection-preventing activity, there has been known, for example, a compound in which a group at i-position of a saccharide is substituted by an N-acyl-N-alkylamino group (Japanese Patent Publication No. 40036/1989) and also a compound in which groups at 1-position and 2-position of a saccharide are each substituted by an N-acyl-N-alkylamino group (Japanese Provisional Patent Publications No. 293991/1986 and No. 311093/1989).

On the other hand, methyl 6-deoxy-2,3-di-O-methyl-6-methylacetamido-α-D-glucopyranoside is disclosed in "Carbohydrate Research", Vol. 45, pp. 65 to 72 (1975), and also methyl 2-N-methylacetamido-2-deoxyglucopyranoside is disclosed in "The Journal of Biochemistry (J. Biochem.), Vol. 78, p. 679 (1975), but no pharmacological activities of said compounds are disclosed.

SUMMARY OF THE INVENTION

The present invention is to provide a novel compound having an excellent infection-preventing activity based on a leukocyte-increasing activity.

The D-glucosamine derivative of the present invention is represented by the formula (I):

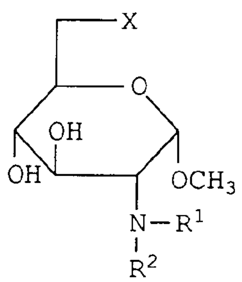

(I)

wherein (1) X is a group represented by the formula:

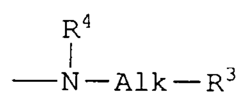

and $R^1$ and $R^2$ are each hydrogen atoms; or
(2) X is hydroxyl group or a group represented by the formula:

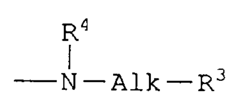

and one of $R^1$ and $R^2$ is a group represented by the formula: -Y-$R^{11}$ or an alkyl group which may have a substituent(s) and the other is hydrogen atom, or one of $R^1$ and $R^2$ is a group represented by the formula: -Y-$R^{11}$ and the other is an alkyl group which may have a substituent(s); Y represents a group represented by the formula: -CO-, -CS- or -$SO_2$-; $R^{11}$ represents an alkyl group which may have a substituent(s), a lower alkoxy group which may have a substituent, an alkenyl group which may have a substituent, an alkynyl group, phenyl group which may have a substituent, a cycloalkyl group, a tricycloalkyl group, a heterocyclic group or an alkylamino group; $R^3$ represents hydrogen atom, phenyl group, phenoxy group, phenylthio group, phenylamino group which may be protected, or a heterocyclic group; $R^4$ represents an alkanoyl group, an alkenoyl group or an alkynoyl group; and Alk represents an alkylene group, provided that a compound in which X is hydroxyl group, one of $R^1$ and $R^2$ is methyl group and the other is acetyl group is excluded.

The objective compound (I) of the present invention which has an excellent leukocyte-increasing activity and an infection-preventing activity is a useful pharmaceutical compound as a leukopenia-treating or preventing agent, an infection-preventing agent or an anti-infectious activity-facilitating agent.

## DESCRIPTION OF THE PREFERRED EMBODIMENT

In the following, the present invention is explained in detail.

In the objective compound (I) of the present invention, as the alkyl group which may have a substituent(s), there may be mentioned an alkyl group which may be substituted by 1 or 2 groups selected from the group consisting of a lower alkoxycarbonyl group, amino group which may be protected, a carboxyalkanoylamino group, an alkylamino group, phenylamino group, carboxyl group, carbamoyl group which may be substituted by an alkyl group, hydroxyl group which may be protected, a monocyclic or bicyclic heterocyclic group containing sulfur atom or nitrogen atom, for example, pyrrolidinyl group, thienyl group, indolyl group, imidazolyl group or pyridyl group, and phenyl group; as the lower alkoxy group which may have a substituent, there may be mentioned a lower alkoxy group which may be substituted by phenyl group; as the alkenyl group which may have a substituent, there may be mentioned an alkenyl group which may be substituted by dimethoxyphenyl group; as the phenyl group which may have a substituent, there may be mentioned phenyl group which may be substituted by a halogen atom; and as the heterocyclic group, there may be mentioned a monocyclic or bicyclic heterocyclic group containing sulfur atom or nitrogen atom described above.

The alkyl group which may have a substituent(s) may be a group which is obtained by removing one carboxyl group from an amino acid, and there may be mentioned, for example, a group which is obtained by removing carboxyl group from a naturally occurring amino acid such as glutamic acid, glutamine, serine, sarcosine, proline, phenylalanine, leucine, isoleucine, glycine, tryptophan, cysteine, histidine, tyrosine and valine, an enantiomer thereof, or a racemic compound thereof.

As the tricycloalkyl group, adamantyl group may be exemplified.

When the alkyl group which may have a substituent(s) is an alkyl group which is substituted by a protected amino group and/or when $R^3$ is a protected phenylamino group, as the protective group of the amino group, there may be mentioned a conventional protective group which can be used as a protective group of amino group, for example, t-butoxycarbonyl group and benzyloxycarbonyl group. There may be also mentioned an alkanoyl group such as acetyl group and butyryl group as the protective group of the amino group.

When the alkyl group which may have a substituent(s) is an alkyl group which is substituted by a protected hydroxyl group, as the protective group of the hydroxyl group, there may be mentioned a conventional protective group which can be used as a protective group of hydroxyl group.

As a pharmaceutically preferred compound, there may be mentioned a compound represented by the formula (I) wherein X is a group represented by the formula:

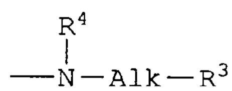

wherein the symbols have the same meanings as above.

As a pharmaceutically more preferred compound, there may be mentioned a compound represented by the formula (I) wherein X is a group represented by the formula:

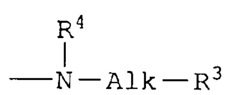

$R^1$ and $R^2$ are both hydrogen atoms; $R^3$ is hydrogen atom, phenyl group, phenoxy group, phenylthio group, phenylamino group which may be protected or a heterocyclic group; and $R^4$ is an alkanoyl group, an alkenoyl group or an alkynoyl group. Among these compounds, a compound wherein $R^3$ is hydrogen atom, phenyl group or phenylamino group which may be protected; and $R^4$ is an alkanoyl group or an alkynoyl group is particularly preferred.

As another pharmaceutically preferred compound, there may be mentioned a compound represented by the formula (I) wherein X is a group represented by the formula:

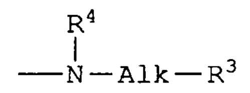

one of $R^1$ and $R^2$ is a group represented by the formula: $-Y-R^{11}$ and the other is hydrogen atom; Y is a group represented by the formula: -CO-, -CS- or $-SO_2-$; $R^{11}$ is an alkyl group which may have a substituent(s), a lower alkoxy group which may have a substituent, an alkenyl group which may have a substituent, an alkynyl group, phenyl group which may have a substituent, a cycloalkyl group, a tricycloalkyl group, a heterocyclic group or an alkylamino group; $R^3$ is hydrogen atom, phenyl group, phenoxy group, phenylthio group, phenylamino group which may be protected or a heterocyclic group; $R^4$ is an alkanoyl group, an alkenoyl group or an alkynoyl group. Among these compounds, a compound wherein $R^3$ is hydrogen atom, phenyl group or phenylamino group which may be protected; and $R^4$ is an alkanoyl group or an alkynoyl group is particularly preferred.

Also as a pharmaceutically preferred compound, there may be mentioned a compound represented by the formula (I) wherein X is a group represented by the formula:

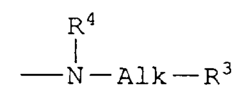

one of $R^1$ and $R^2$ is a group represented by the formula: $-Y-R^{11}$ and the other is hydrogen atom; Y is a group represented by the formula: -CO-; $R^{11}$ is an alkyl group which may have a substituent(s); $R^3$ is hydrogen atom or phenyl group; and $R^4$ is an alkanoyl group or an alkynoyl group. Among these compounds, (i) a compound wherein $R^{11}$ is an alkyl group which may be substituted by 1 or 2 groups selected from the group consisting of a lower alkoxycarbonyl group, amino group which may be protected, a carboxy-alkanoylamino group, an alkylamino group, phenylamino group, carboxyl group, carbamoyl group, hydroxyl group and phenyl group, or (ii) a compound wherein $R^{11}$ is a group obtained by removing one carboxyl group from a naturally occurring amino acid or an enantiomer thereof is particularly preferred.

Pharmaceutically, as most preferred compound, there may be mentioned a compound represented by the formula (I) wherein X is a group represented by the formula:

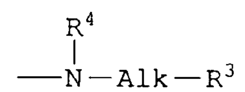

one of $R^1$ and $R^2$ is a group represented by the formula: $-Y-R^{11}$ and the other is hydrogen atom; Y is a group represented by the formula: -CO-; $R^{11}$ is an alkyl group which may be substituted by amino group which may be protected; $R^3$ is hydrogen atom or phenyl group; and $R^4$ is an alkanoyl group or an alkynoyl group.

The objective compound (I) of the present invention includes an optical isomer thereof and a racemate thereof.

The objective compound (I) of the present invention can be used for medical/pharmaceutical uses in the free form or in the form of pharmaceutically acceptable salt thereof. As such a salt, there may be mentioned, for example, a metal salt such as an alkali metal salt and an alkaline earth metal salt, an ammonium salt, an organic amine salt such as a tri-lower alkylamine, an amino acid salt, an organic acid salt such as succinate, maleate, tartrate and methanesulfonate, and a mineral acid salt such as hydrochloride, sulfate and nitrate.

The objective compound (I) of the present invention can be orally and parenterally administered, and can be used in combination with a carrier or a diluent which is suitable for oral or parenteral administration, as a pharmaceutical preparation. The pharmaceutical preparation can be a solid preparation such as a tablet, capsule and powder, or a liquid preparation such as a solution, suspension and emulsion. It can be also administered parenterally in the form of injection.

The dose of the objective compound (I) of the present invention, varying depending on the age, the body weight and the condition of patient, or the severity of the disease, is 1 to 500 mg/kg/day, preferably 10 to 100 mg/kg/day for the oral administration, and 0.1 to 500 mg/kg/day, preferably 1 to 100 mg/kg/day for the parenteral administration.

According to the present invention, (1) among the objective compound (I), a D-glucosamine derivative represented by the formula (I-a):

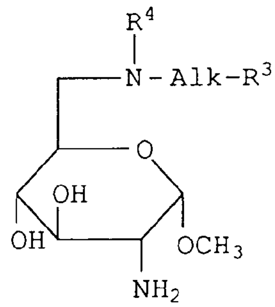

(I-a)

wherein the symbols have the same meanings as above, can be prepared by condensing a D-glucosamine compound represented by the formula (II) :

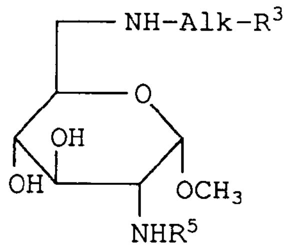

(II)

wherein $NHR^5$ represents amino group which may be protected and the other symbols have the same meanings as above,
or a salt thereof with a compound represented by the formula (III):

$R^4$-OH (III)

wherein $R^4$ has the same meaning as above, a salt thereof, or a reactive derivative thereof, and if necessary, by removing the protective group.

(2) Among the objective compound (I) of the present invention, a D-glucosamine derivative represented by the formula (I-b):

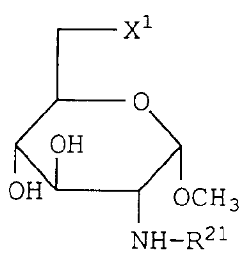

(I-b)

wherein $R^{21}$ represents an alkyl group which may have a substituent(s); $X^1$ represents hydroxyl group or a group represented by the formula:

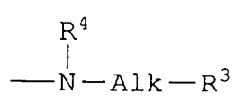

where the symbols have the same meanings as above, can be prepared by alkylating a D-glucosamine compound represented by the formula (IV):

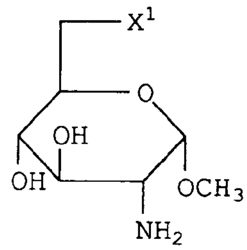

(IV)

wherein the symbols have the same meanings as above, or a salt thereof.

(3) Among the objective compound (I) of the present invention, a D-glucosamine derivative represented by the formula (I-c):

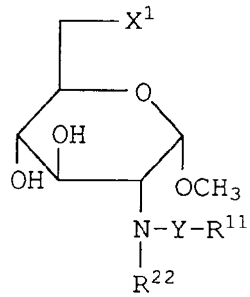

(I-c)

wherein $R^{22}$ represents hydrogen atom or an alkyl group which may have a substituent(s); and the other symbols have the same meanings as above, provided that when $X^1$ is hydroxyl group and $R^{22}$ is methyl group, $R^{11}$-Y- is not acetyl group,
can be prepared by condensing a compound represented by the formula (V):

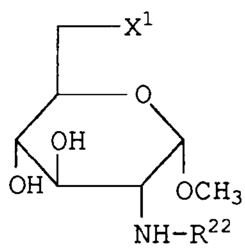

(V)

wherein the symbols have the same meanings as above, or a salt thereof with a compound represented by the formula (VI):

$R^{11}$-Y-OH (VI)

wherein the symbols have the same meanings as above, a salt thereof or a reactive derivative thereof.

In the process (1), when $NHR^5$ is a protected amino group, as the protective group of the amino group, a conventional protective group which can be used as a protective group of amino group can be used, and there may be preferably exemplified benzyloxycarbonyl group and t-butoxycarbonyl group.

As the salt of D-glucosamine compound (II), (IV) or (V) (hereinafter, referred to as a "D-glucosamine compound") in the processes (1) to (3), there may be used a conventional salt such as a mineral acid salt including hydrochloride and an organic acid salt including fumarate, maleate, malate and oxalate.

In the processes (1) and (3), the condensation reaction of the "D-glucosamine compound" or a salt thereof with the compound (III) or (VI), or a salt thereof can be carried out in an appropriate solvent in the presence of a dehydrating agent.

As the salt of the compound (III) or (VI), there may be used a conventional salt such as an alkali metal salt and an alkali earth metal salt, and the salt may be preferably used as a free compound for the reaction with the D-glucosamine compound.

As the dehydrating agent, there may be mentioned a conventional dehydrating agent such as 1,3-dicyclohexyl-carbodiimide, 1-ethyl-3-(3'-dimethylaminopropyl)carbodiimide and carbonyldiimidazole.

In the processes (1) and (3), the condensation reaction of the "D-glucosamine compound" or a salt thereof

with the reactive derivative of the compound (III) or (VI) can be carried out in an appropriate solvent in the presence or absence of an acid acceptor.

As the reactive derivative of the compound (III) or (VI), there may be used a conventional one which is used for a condensation reaction, for example, such as an acid halide, a mixed acid anhydride and an active ester. Also as the reactive derivative of the compound (VI), there may be mentioned an isocyanate or an isothiacyanate which reacts with the compound (V) to form the compound (I-c) in which $R^{11}$ is an alkylamino group, or a halogenoformic acid ester which reacts with the compound (V) to form the compound (I-c) in which $R^{11}$ is a lower alkoxy group which may have a substituent.

As the acid acceptor, there may be mentioned an alkali metal carbonate such as potassium carbonate and sodium carbonate; an alkali metal hydrogencarbonate such as potassium hydrogencarbonate and sodium hydrogencarbonate; an alkali metal hydroxide such as sodium hydroxide and potassium hydroxide; a tertiary amine such as a tri lower alkylamine including triethylamine and diisopropylethylamine, 1,4-diazabicyclo[2,2,2]octane, 1,5-diazabicyclo[4,3,0]-5-nonene and 1,8-diazabicyclo[5,4,0]-7-undecene; and an aromatic amine such as pyridine, dimethylaminopyridine, lutidine, collidine and dimethylaniline.

As the solvent for the reaction in the processes (1) and (3), an inert solvent which does not affect the condensation reaction is used, and there may be used, for example, water; a halogenated solvent such as chloroform, dichloromethane and 1,2-dichloroethane; an ether solvent such as tetrahydrofuran, dioxane and diethyl ether; an aromatic hydrocarbon such as benzene, toluene and xylene; an amide solvent such as dimethylformamide and dimethylacetamide; an aromatic amine solvent such as pyridine, lutidine and collidine; an ester solvent such as ethyl acetate and butyl acetate; a ketone solvent such as acetone and methyl ethyl ketone; and a mixture solvent thereof. The condensation reaction can preferably be carried out from under cooling to at the boiling point of the solvent, for example at -30 °C to 150 °C, particularly at -10 °C to room temperature.

In the process (2), the alkylating reaction of the D-glucosamine derivative (IV) can be carried out, for example, by (2-a) reductively reacting the compound (IV) with a compound represented by the formula (VII) :

$$R^{24}\text{-CHO} \qquad \text{(VII)}$$

wherein $R^{24}$ represents hydrogen atom or an alkyl group which may have a substituent(s),

in a solvent in the presence of a reducing agent or by a catalytic reduction, or by (2-b) condensing the compound (IV) with a compound represented by the formula (VIII):

$$R^{21}\text{-}Z^{1} \qquad \text{(VIII)}$$

wherein $Z^1$ represents a reactive residue and the other symbols have the same meanings above,

in an appropriate solvent or without a solvent in the presence or absence of an acid acceptor and a reaction-accelerator. As $Z^1$ of the compound (VIII), a conventional eliminating group can be used, and there may be mentioned, for example, a halogen atom such as chlorine atom, bromine atom and iodine atom, methanesulfonyloxy group, benzenesulfonyloxy group and toluenesulfonyloxy group.

In the reaction (2-a) for reductive alkylation, as the reducing agent, a conventional metal hydride can be used, and there may be mentioned, for example, sodium borohydride, lithium aluminum hydride, sodium cyanoborohydride, aluminum diisobutylhydride and sodium dihydrobis(2-methoxy-ethoxy)aluminum. The catalytic reduction can be carried out according to a conventional method, for example, under hydrogen flow by using palladium-carbon or platinum oxide as a catalyst.

As the solvent, an inert solvent which does not affect the reaction is used, and there may be mentioned, for example, water; an alcohol such as ethanol and methanol; an aromatic hydrocarbon such as benzene and toluene; acetic acid; and an ether solvent such as tetrahydrofuran.

The reaction temperature may be from under cooling to under heating, preferably -30 to 100 °C, particularly 0 to 80 °C.

The reaction (2-b) for alkylating the compound (IV) by condensation can be carried out according to a conventional method. As the acid acceptor, the acid acceptor which is exemplified in the above processes (1) and (3) can be used. As the reaction-accelerator, there may be mentioned, for example, sodium iodide. As the solvent, an inert solvent which does not affect the reaction is used, and there may be used an alcohol solvent such as methanol, ethanol, propanol and ethylene glycol; a halogenated solvent such as chloroform, dichloromethane and 1,2-dichloroethane; an ether solvent such as tetrahydrofuran, dioxane and 1,2-dimethoxyethane; an aromatic hydrocarbon such as benzene, toluene and xylene; an amide solvent such as dimethylformamide and dimethylacetamide; dimethylsulfoxide; and a mixed solvent thereof. The condensation reaction may be carried out, for example at 0 to 200 °C, preferably at 70 to 150 °C.

The above compound can be mutually converted, and for example, a compound represented by the formula (I-b) can be also prepared by condensing the compound (IV) or a salt thereof with a compound represented by the formula (VI-a) :

$$R^{23}\text{-COOH} \qquad \text{(VI-a)}$$

wherein $R^{23}$ represents an alkyl group which may have a substituent(s),

a salt thereof or a reactive derivative thereof to have a compound represented by the formula (I-d) :

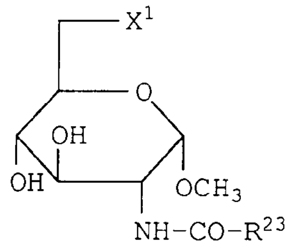

(I-d)

wherein the symbols have the same meanings as above, and then by reducing the obtained compound.

The condensation reaction of the compound (IV) with the compound (IV-a) can be carried out in the same manner as in the process (1) or (3).

The reduction reaction of the compound (I-d) can be carried out in the same manner as in the process (2) in an appropriate solvent in the presence of a reducing agent or by a catalytic reduction according to a conventional method.

The starting compound (II) of the present invention is a novel compound and can be prepared by reacting a compound represented by the formula (IX) :

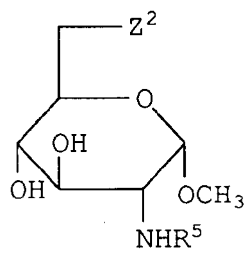

(IX)

wherein $Z^2$ represents an eliminating group and the other symbols have the same meanings as above, with an amine compound represented by the formula (X) :

$$H_2N\text{-Alk-}R^3 \qquad (X)$$

wherein the symbols have the same meanings as above.

As the eliminating group $Z^2$, a conventional eliminating group can be used, and there may be mentioned, for example, methanesulfonyloxy group and p-toluenesulfonyloxy group.

The substitution reaction may be carried out in the same manner as in the above process (2-a).

In the specification of the present invention, as the alkyl group and the alkylene group, there may be mentioned an alkyl group having 1 to 30 carbon atoms and an alkylene group having 1 to 30 carbon atoms, respectively, and as the alkenyl group and the alkynyl group, there may be mentioned an alkenyl group having 2 to 30 carbon atoms and an alkynyl group having 2 to 30 carbon atoms, respectively. As the alkanoyl group, there may be mentioned an alkanoyl group having 2 to 30 carbon atoms, and as the alkenoyl group and the alkynoyl group, there may be mentioned an alkenoyl group having 3 to 30 carbon atoms and an alkynoyl group having 3 to 30 carbon atoms, respectively. As the lower alkoxy group, there may be mentioned an alkoxy group having 1 to 6 carbon atoms. As the cycloalkyl group, there may be mentioned a cycloalkyl group having 3 to 6 carbon atoms, particularly a cyclohexyl group.

In the specification of the present invention, the alkyl group, the alkylene group, the alkenyl group, the alkynyl group, the alkanoyl group, the alkenoyl group, the alkynoyl group and the lower alkoxy group each include straight ones and branched ones.

## EXAMPLES

### Example 1

To 10.45 g of methyl 2,6-dideoxy-2-benzyloxycarbonylamino-6-(4-phenylbutylamino)-α-D-glucopyranoside dissolved in 200 ml of tetrahydrofuran was added an aqueous solution containing 6.91 g of potassium carbonate dissolved in 50 ml of water and 7.60 g of stearoyl chloride dissolved in 20 ml of tetrahydrofuran was

added dropwise to the mixture while stirring under cooling, followed by stirring for 2 hours. To the reaction solution were added 2 l of ethyl acetate and 1 l of water and an insoluble material was removed by filtration. The organic layer was taken, washed with water and dried, and the solvent was removed by evaporation. The residue was purified by silica gel column chromatography (solvent; ethyl acetate) to give 14.04 g of methyl 2,6-dideoxy-2-benzyloxycarbonyamino-6-[N-stearoyl-N-(4-phenylbutyl)amino]-α-D-glucopyranoside.
IR (nujol) $cm^{-1}$: 3600-3100, 1720, 1620
FABMS (m/z): 725 ($MH^+$)

Examples 2 to 5

The corresponding starting compounds were treated in the same manner as in Example 1 to give compounds shown in Table 1.

Table 1

| Example | 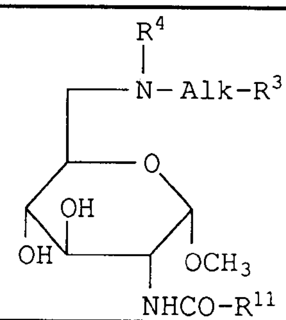 | | | |
|---|---|---|---|---|
| | $R^{11}$ | $R^3$-Alk- | $R^4$ | Physical properties |
| 2 | 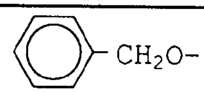 | $H-(CH_2)_{18}-$ | $CH_3(CH_2)_{10}CO-$ | IR (neat) $cm^{-1}$: 3600-3100, 1700, 1650<br>FABMS (m/z): 761 ($MH^+$) |
| 3 | 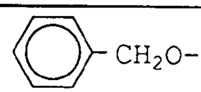 | 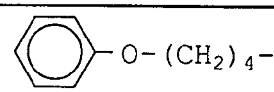 | $CH_3(CH_2)_{16}CO-$ | IR (neat) $cm^{-1}$: 3600-3100, 1720, 1620<br>FABMS (m/z): 741 ($MH^+$) |
| 4 | 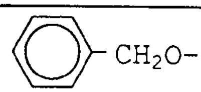 | 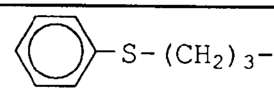 | $CH_3(CH_2)_{16}CO-$ | IR (neat) $cm^{-1}$: 3600-3100, 1720, 1620<br>FABMS (m/z): 743 ($MH^+$) |
| 5 | $(CH_3)_3CO-$ | 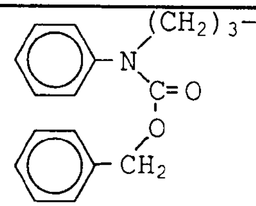 | $CH_3(CH_2)_{16}CO-$ | IR (neat) $cm^{-1}$: 3600-3100, 1710, 1620<br>FABMS (m/z): 826 ($MH^+$) |

Example 6

To 14.04 g of methyl 2,6-dideoxy-2-benzyloxycarbonylamino-6-[N-stearoyl-N-(4-phenylbutyl)amino]-α-D-glucopyranoside dissolved in 300 ml of methanol was added 10.0 g of 10 % palladium-carbon to carry out a catalytic hydrogen reduction at room temperature under atmospheric pressure of hydrogen. After 4 hours, the catalyst was removed by filtration and then methanol was removed by evaporation. The residue was purified by silica gel column chromatography (solvent; chloroform:methanol = 5 : 1) to give 11.25 g of methyl 2,6-dideoxy-2-amino-6-[N-stearoyl-N-(4-phenylbutyl)amino]-α-D-glucopyranoside.
IR (nujol) $cm^{-1}$: 3600-3100, 1640
FABMS (m/z): 591 ($MH^+$)

Examples 7 to 9

The corresponding starting compounds were treated in the same manner as in Example 6 to give compounds shown in Table 2.

Table 2

| Example | 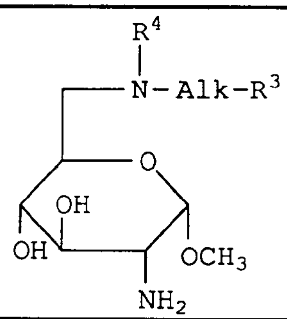 | | |
|---|---|---|---|
| | $R^3$-Alk- | $R^4$ | Physical properties |
| 7 | H-$(CH_2)_{18}$- | $CH_3(CH_2)_{10}CO$- | IR(neat) $cm^{-1}$:3600-3100, 1650<br>FABMS(m/z): 649 [$(M+Na)^+$] |
| 8 | 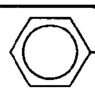-O-$(CH_2)_4$- | $CH_3(CH_2)_{16}CO$- | IR(neat) $cm^{-1}$:3600-3100, 1620<br>FABMS(m/z): 607 ($MH^+$) |
| 9 | 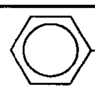-S-$(CH_2)_3$- | $CH_3(CH_2)_{16}CO$- | IR(neat) $cm^{-1}$:3600-3100, 1620<br>FABMS(m/z): 609 ($MH^+$) |

Example 10

To 2.02 g of methyl 2,6-dideoxy-2-t-butoxycarbonylamino-6-{N-stearoyl-N-[3-(N-phenyl-N-benzyloxycarbonylamino)-propyl]amino}-α-D-glucopyranoside dissolved in 10 ml of dichloromethane was added 2 ml of trifluoroacetic acid, followed by stirring at room temperature for 3 hours. To the reaction solution was added 10 % sodium hydroxide aqueous solution and then were added 200 ml of chloroform and 70 ml of water. The organic layer was taken, washed with water and dried, and the solvent was removed by evaporation. The residue was purified by silica gel column chromatography (solvent; chloroform:methanol=15:1) to give 1.72 g of methyl 2,6-dideoxy-2-amino-6-{N-stearoyl-N-[3-(N-phenyl-N-benzyloxycarbonylamino)propyl}-α-D-glucopyranoside.
IR (neat) $cm^{-1}$: 3600-3100, 1710, 1640
FABMS (m/z): 726 ($MH^+$)

Example 11

Methyl 2,6-dideoxy-2-amino-6-{N-stearoyl-N-[3-(N-phenyl-N-benzyloxycarbonylamino)propyl]amino}-α-D-glucopyranoside was treated in the same manner as in Example 6 to give methyl 2,6-dideoxy-2-amino-6-{N-stearoyl-N-[3-(phenylamino) propyl]amino}-α-D-glucopyranoside.
IR (nujol) $cm^{-1}$: 3600-3100, 1620
FABMS (m/z): 592 ($MH^+$)

Example 12

To 478 mg of methyl 2,6-dideoxy-2-amino-6-[N-stearoyl-N-(4-phenylbutyl)amino]-α-D-glucopyranoside dissolved in 10 ml of tetrahydrofuran was added 225 mg of potassium carbonate dissolved in 2 ml of water while cooling with ice under stirring and then 269 mg of stearoyl chloride dissolved in 2 ml of tetrahydrofuran was added to the mixture, followed by stirring for 1 hour. To the reaction solution were added 50 ml of ethyl acetate and 10 ml of water, the organic layer was taken, washed with water and dried, and the solvent was removed by evaporation. The residue was purified by silica gel column chromatography (solvent; chloroform:methanol=20:1) to give 645 mg of methyl 2,6-dideoxy-2-stearoylamino-6-[N-stearoyl-N-(4-phenylbutyl) amino]-α-D-glucopyranoside.
m.p.: 51 to 52 °C
IR (nujol) $cm^{-1}$: 3600-3100, 1640
FABMS (m/z): 858 ($MH^+$)

Examples 13 to 35

The corresponding starting compounds were treated in the same manner as in Example 12 to give compounds shown in Tables 3 to 5.

Table 3

| Example | 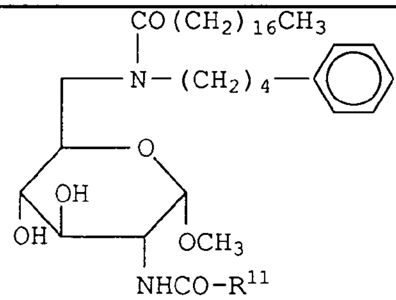 | |
|---|---|---|
| | $R^{11}$ | Physical properties |
| 13 | $CH_3(CH_2)_8-$ | IR(neat)$cm^{-1}$:3600-3100, 1630<br>FABMS(m/z): 746 ($MH^+$) |
| 14 | $CH_2=CH(CH_2)_8-$ | IR(neat)$cm^{-1}$:3600-3100, 1640<br>FABMS(m/z): 757 ($MH^+$) |
| 15 | $CH_3-$ | IR(neat)$cm^{-1}$:3600-3100, 1640<br>FABMS(m/z): 633 ($MH^+$) |
| 16 | 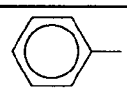 | IR(neat)$cm^{-1}$:3600-3100, 1640<br>FABMS(m/z): 695 ($MH^+$) |
| 17 | 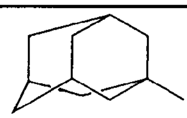 | IR(neat)$cm^{-1}$:3600-3100, 1640<br>FABMS(m/z): 753 ($MH^+$) |
| 18 | 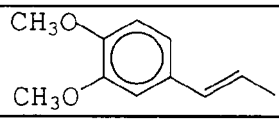 | m.p.: 50-51 °C |
| 19 | $(CH_3CH_2)_2CH-$ | IR(neat)$cm^{-1}$:3600-3100, 1630<br>FABMS(m/z): 689 ($MH^+$) |
| 20 | 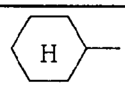 | m.p.: 74-76 °C |
| 21 | $(CH_3)_3C-$ | IR(neat)$cm^{-1}$:3600-3100, 1640<br>FABMS(m/z): 675 ($MH^+$) |
| 22 | $CH_3OCO(CH_2)_4-$ | IR(nujol)$cm^{-1}$:3600-3100, 1740, 1640<br>FABMS(m/z): 755 [$(M+Na)^+$] |

<u>Table 4</u>

| Example | 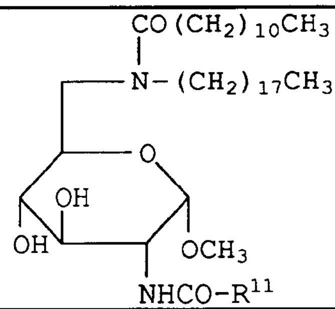 | |
|---|---|---|
| | $R^{11}$ | Physical properties |
| 23 | $CH_3-$ | IR(neat)$cm^{-1}$:3600-3100, 1650<br>FABMS(m/z): 669 ($MH^+$) |
| 24 | $CH_3(CH_2)_{10}-$ | m.p.: 69-70 °C |
| 25 | $CH_3(CH_2)_{16}-$ | m.p.: 78-79 °C |
| 26 | $CH_3(CH_2)_4-$ | m.p.: 75-77 °C |
| 27 | 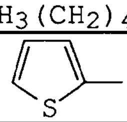 | IR(neat)$cm^{-1}$:3600-3100, 1650<br>FABMS(m/z): 737 ($MH^+$) |
| 28 | 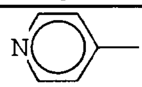 | IR(neat)$cm^{-1}$:3600-3100, 1640<br>FABMS(m/z): 732 ($MH^+$) |
| 29 | 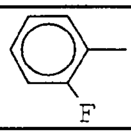 | IR(neat)$cm^{-1}$:3600-3100, 1650, 1620<br>FABMS(m/z): 749 ($MH^+$) |
| 30 | $(CH_3)_2CH-$ | IR(neat)$cm^{-1}$:3600-3100, 1650, 1630<br>FABMS(m/z): 697 ($MH^+$) |
| 31 | $CH_3CH_2-$ | IR(neat)$cm^{-1}$:3600-3100, 1650, 1620<br>FABMS(m/z): 683 ($MH^+$) |
| 32 | $CH_3CH_2CH_2-$ | IR(neat)$cm^{-1}$:3600-3100, 1640, 1620<br>FABMS(m/z): 697 ($MH^+$) |

Table 5

| Example | 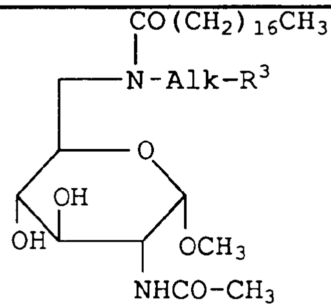 | |
|---|---|---|
| | $R^3$-Alk- | Physical properties |
| 33 | 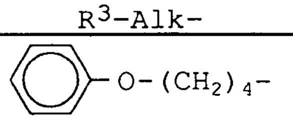 | IR(neat)$cm^{-1}$:3600-3100, 1640<br>FABMS(m/z): 649 ($MH^+$) |
| 34 | 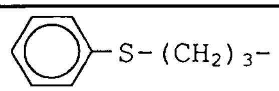 | IR(neat)$cm^{-1}$:3600-3100, 1640<br>FABMS(m/z): 651 ($MH^+$) |
| 35 | 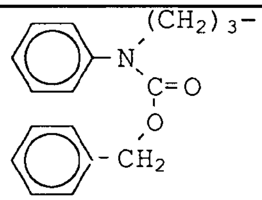 | IR(neat)$cm^{-1}$:3600-3100, 1710, 1650<br>FABMS(m/z): 768 ($MH^+$) |

Example 36

To 1.54 g of methyl 2,6-dideoxy-2-amino-6-[N-stearoyl-N-(4-phenylbutyl)amino]-α-D-glucopyranoside and 0.75 g of N-t-butoxycarbonyl-L-leucine dissolved in 25 ml of tetrahydrofuran were added 0.49 g of 95 % diethyl cyanophosphate and 0.55 g of triethylamine under cooling with ice, followed by stirring for 1 hour. The mixture was further stirred at room temperature for 45 minutes and tetrahydrofuran was removed by evaporation. The residue was dissolved in ethyl acetate and the ethyl acetate layer was washed with water and dried. Then, the solvent was removed by evaporation. The residue was purified by silica gel column chromatography (solvent; hexane : ethyl acetate = 1 : 3) to give 1.70 g of methyl 2,6-dideoxy-2-[N-(t-butoxycarbonyl)-L-leucyl]amino-6-[N-stearoyl-N-(4-phenylbutyl)amino]-α-D-glucopyranoside.
IR (neat) $cm^{-1}$: 3600-3100, 1700, 1650, 1630
FABMS (m/z): 804 ($MH^+$)

Example 37 to 41

The corresponding starting compounds were treated in the same manner as in Example 36 to give compounds shown in Table 6.

Table 6

| Example | 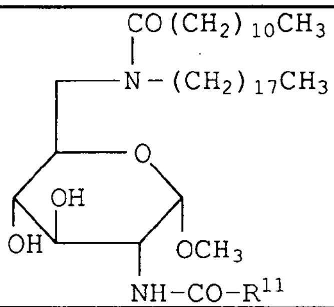 | |
|---|---|---|
| | $R^{11}$ | Physical properties |
| 37 | 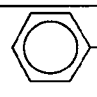 $-CH_2OCONHCH_2-$ | IR(nujol) $cm^{-1}$: 3600-3100, 1720, 1650<br>FABMS(m/z): 818 ($MH^+$) |
| 38 | $(CH_3)_2CHCH-$<br>(L)<br>$NHOCOC(CH_3)_3$ | IR(nujol) $cm^{-1}$: 3600-3100, 1700, 1650<br>FABMS(m/z): 826 ($MH^+$) |
| 39 | $CH_3CH-$<br>(L)<br>$NHOCOC(CH_3)_3$ | IR(nujol) $cm^{-1}$: 3600-3100, 1720, 1650<br>FABMS(m/z): 798 ($MH^+$) |
| 40 | $(CH_3)_2CHCH_2CH-$<br>(D)<br>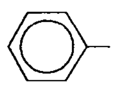 $-CH_2OCONH$ | IR(nujol) $cm^{-1}$: 3600-3100, 1720, 1650<br>FABMS(m/z): 874 ($MH^+$) |
| 41 | $(CH_3)_2CHCH_2CH-$<br>(L)<br>$(CH_3)_3COCONH$ | IR(neat) $cm^{-1}$: 3600-3100, 1620<br>FABMS(m/z): 592 ($MH^+$) |

Example 42

To 1.70 g of methyl 2,6-dideoxy-2-[N-(t-butoxycarbonyl)-L-leucyl]amino-6-[N-stearoyl-N-(4-phenylbutyl)amino]-α-D-glucopyranoside dissolved in 10 ml of dichloromethane was added 2 ml of trifluoroacetic acid, followed by stirring at room temperature for 5 hours. To the reaction solution was added 10 % sodium hydroxide aqueous solution and then were added 150 ml of chloroform and 50 ml of water. The organic layer was taken, washed with water and dried, and the solvent was removed by evaporation. The residue was purified by silica gel column chromatography (solvent; chloroform : methanol = 10 : 1) to give 1.47 g of methyl 2,6-dideoxy-2-(L-leucyl)amino-6-[N-stearoyl-N-(4-phenylbutyl)amino]-α-D-glucopyranoside.
IR (nujol) $cm^{-1}$: 3600-3100, 1650
FABMS (m/z): 705 ($MH^+$)

Examples 43 to 45

The corresponding starting compounds were treated in the same manner as in Example 42 to give compounds shown in Table 7.

Table 7

| Example | 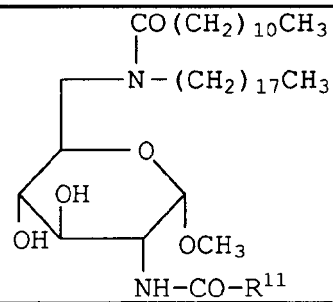 | |
|---|---|---|
| | $R^{11}$ | Physical properties |
| 43 | $(CH_3)_2CHCH_2CH-$<br>\| (L)<br>$NH_2$ | IR(neat)$cm^{-1}$:3600-3100, 1650<br>FABMS(m/z): 740($MH^+$) |
| 44 | $CH_3CH-$<br>\| (L)<br>$NH_2$ | IR(neat)$cm^{-1}$:3600-3100, 1650<br>FABMS(m/z): 698($MH^+$) |
| 45 | $(CH_3)_2CHCH-$<br>\| (L)<br>$NH_2$ | IR(neat)$cm^{-1}$:3600-3100, 1640<br>FABMS(m/z): 726($MH^+$) |

Examples 46 to 47

The corresponding starting compounds were treated in the same manner as in Example 6 to give compounds shown in Table 8.

Table 8

| Example | 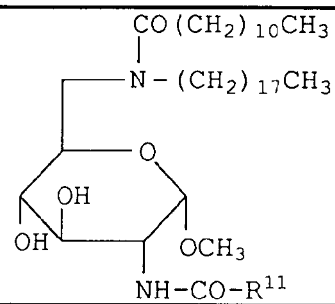 | |
|---|---|---|
| | $R^{11}$ | Physical properties |
| 46 | $(CH_3)_2CHCH_2CH-$<br>\| (D)<br>$NH_2$ | IR(neat)$cm^{-1}$:3600-3100, 1650<br>FABMS(m/z): 740($MH^+$) |
| 47 | $NH_2CH_2-$ | IR(neat)$cm^{-1}$:3600-3100, 1650<br>FABMS(m/z): 684($MH^+$) |

Example 48

To 400 mg of methyl 2,6-dideoxy-2-(L-leucyl)amino-6-[N-stearoyl-N-(4-phenylbutyl)amino]-α-D-glucopyranoside dissolved in 10 ml of ethanol was added 56 mg of succinic anhydride, followed by stirring at room temperature for 1 hour. Ethanol was removed by evaporation and the residue was purified by silica gel column chromatography (solvent; chloroform : methanol = 10 : 1) to give 450 mg of methyl 2,6-dideoxy-2-[N-(carboxypropionyl)-L-leucyl]amino-6-[N-stearoyl-N-(4-phenylbutyl)amino]-α-D-glucopyranoside.
IR (nujol) $cm^{-1}$: 3600-3100, 1720, 1650
FABMS (m/z): 826 [$(M+Na^{+})$]

Example 49

The corresponding starting compound was treated in the same manner as in Example 48 to give methyl 2,6-dideoxy-2-[(N-carboxypropionyl)-L-leucyl]amino-6-[N-lauroyl-N-octadecylamino]-α-D-glucopyranoside.
IR (neat) $cm^{-1}$: 3600-3100, 1720, 1650
FABMS (m/z): 840 ($MH^{+}$)

Example 50

To 1.50 g of methyl 2,6-dideoxy-2-amino-6-[N-stearoyl-N-(4-phenylbutyl)amino]-α-D-glucopyranoside dissolved in 30 ml of tetrahydrofuran was added 1.25 g of potassium carbonate dissolved in 15 ml of water, and to the mixture was added dropwise 1.90 g of octanesulfonyl chloride dissolved in 20 ml of tetrahydrofuran under cooling with ice, followed by stirring for 3 hours. To the reaction mixture was added 150 ml of ethyl acetate and the organic layer was washed with water and dried. Then the solvent was removed by evaporation. The residue was purified by silica gel column chromatography (solvent; hexane : ethyl acetate = 1 : 3) to give 1.56 g of methyl 2,6-dideoxy-2-octanesulfonylamino-6-[N-stearoyl-N-(4-phenylbutyl)amino]-α-D-glucopyranoside.
IR (neat) $cm^{-1}$: 3600-3100, 1620
FABMS (m/z): 767 ($MH^{+}$)

Example 51

The corresponding starting compound was treated in the same manner as in Example 50 to give methyl 2,6-dideoxy-2-octanesulfonylamino-6-(N-lauroyl-N-octadecylamino)-α-D-glucopyranoside.
IR (neat) $cm^{-1}$: 3600-3100, 1620
FABMS (m/z): 803 ($MH^{+}$)

Example 52

The corresponding starting compound was treated in the same manner as in Example 50 to give methyl 2,6-dideoxy-2-methanesulfonylamino-6-(N-lauroyl-N-octadecylamino)-α-D-glucopyranoside.
IR (neat) $cm^{-1}$: 3600-3100, 1620
FABMS (m/z): 705 ($MH^{+}$)

Example 53

To 300 mg of methyl 2,6-dideoxy-2-amino-6-[N-stearoyl-N-(4-phenylbutyl) amino]-α-D-glucopyranoside and 226 mg of 4-phenylbutanal were dissolved in 8 ml of methanol was added 2 ml of acetic acid. The reaction mixture was hydrogenated over 10 mg of platinum oxide at room temperature under atmospheric pressure of hydrogen. After 8 hours, the catalyst was removed and the solvent was removed by evaporation. The residue was purified by silica gel column chromatography (solvent; chloroform : methanol = 10 : 1) to give 320 mg of methyl 2,6-dideoxy-2-(4-phenylbutyl)amino-6-[N-stearoyl-N-(4-phenylbutyl)amino]-α-D-glucopyranoside.
IR (neat) $cm^{-1}$: 3600-3100, 1620
FABMS (m/z): 723 ($MH^{+}$)

Example 54

The corresponding starting compound was treated in the same manner as in Example 53 to give methyl 2,6-dideoxy-2-octadecylamino-6-(N-lauroyl-N-octadecylamino]-α-D-glucopyranoside.
IR (neat) $cm^{-1}$: 3600-3100, 1650
FABMS (m/z): 879 ($MH^{+}$)

Example 55

The corresponding starting compound was treated in the same manner as in Example 53 to give methyl

2,6-dideoxy-2-propylamino-6-(N-lauroyl-N-octadecylamino]-α-D-glucopyranoside.
IR (neat) $cm^{-1}$: 3600-3100, 1650
FABMS (m/z): 669 ($MH^+$)

Example 56

The corresponding starting compound was treated in the same manner as in Example 12 to give methyl 2,6-dideoxy-2-[N-(4-phenylbutyl)-N-stearoylamino]-6-[N-(4-phenylbutyl)-N-stearoylamino]-α-D-glucopyranoside.
IR (neat) $cm^{-1}$: 3600-3100, 1650, 1620
FABMS (m/z): 1011 [($M+Na^+$)]

Examples 57 to 61

The corresponding starting compounds were treated in the same manner as in Example 12 to give compounds shown in Table 9.

Table 9

| Example | 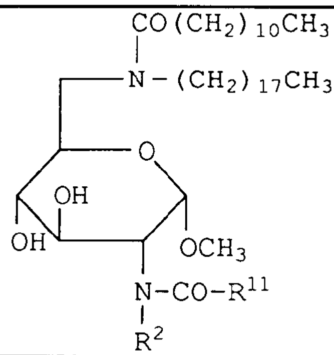 | | |
|---|---|---|---|
| | $R^{11}$ | $R^2$ | Physical properties |
| 57 | $CH_3(CH_2)_{10}-$ | $CH_3(CH_2)_{17}-$ | IR(neat) $cm^{-1}$:3600-3100, 1650, 1620<br>FABMS (m/z) : 1083 [$(M+Na)^+$] |
| 58 | $CH_3-$ | $CH_3(CH_2)_{17}-$ | IR(neat) $cm^{-1}$:3600-3100, 1650, 1620<br>FABMS (m/z) : 943 [$(M+Na)^+$] |
| 59 | $CH_3-$ | $CH_3(CH_2)_2-$ | IR(neat) $cm^{-1}$:3600-3100, 1650, 1620<br>FABMS (m/z) : 711 ($MH^+$) |
| 60 | $CH_3(CH_2)_2-$ | $CH_3(CH_2)_2-$ | IR(neat) $cm^{-1}$:3600-3100, 1650, 1620<br>FABMS (m/z) : 743 ($MH^+$) |
| 61 | $CH_3O-$ | $CH_3(CH_2)_2-$ | IR(neat) $cm^{-1}$:3600-3100, 1700, 1650<br>FABMS (m/z) : 727 ($MH^+$) |

Example 62

To 450 mg of nonadecanoic acid dissolved in 20 ml of toluene were added 0.21 ml of triethylamine and 0.32 ml of diphenylphosphoric acid azide, followed by heat-reflux. After 6 hours, to the mixture was added 1.02 g of methyl 2,6-dideoxy-2-amino-6-(N-lauroyl-N-octadecylamino)-α-D-glucopyranoside dissolved in 5 ml of toluene, followed by reflux overnight. After cooling, the organic layer was washed with water and dried, and the solvent was removed by evaporation. The residue was purified by silica gel column chromatography (solvent; hexane : ethyl acetate = 1 : 4) to give 1.11 g of methyl 2,6-dideoxy-2-octadecylaminocarbonylamino-6-(N-lauroyl-N-octadecylamino)-α-D-glucopyranoside.

m.p.: 89 to 90 °C

Example 63

To 500 mg of methyl 2,6-dideoxy-2-propylamino-6-(N-lauroyl-N-octadecyl)amino-α-D-glucopyranoside dissolved in 15 ml of dichloromethane was added 43 mg of methyl isocyanate, followed by stirring at room temperature for 40 minutes. The solvent was removed by evaporation and the residue was purified by silica gel column chromatography (solvent; chloroform : methanol = 25 : 1) to give 430 mg of methyl 2,6-dideoxy-2-(N-propyl-N-methylaminocarbonyl)amino-6-(N-lauroyl-N-octadecylamino-α-D-glucopyranoside.
IR (neat) $cm^{-1}$: 3600-3100, 1630
FABMS (m/z): 726 ($MH^+$)

Example 64

The corresponding starting compound and methyl isothiocyanate were treated in the same manner as in Example 63 to give methyl 2,6-dideoxy-2-[N-propyl-N-(methylaminothiocarbonyl)amino]-6-(N-lauroyl-N-octadecylamino-α-D-glucopyranoside.
IR (neat) $cm^{-1}$: 3600-3100, 1620
FABMS (m/z): 742 ($MH^+$)

Example 65

The corresponding starting compound was treated in the same manner as in Example 6 to give methyl 2,6-dideoxy-2-acetylamino-6-[N-stearoyl-N-(3-phenylaminopropyl)amino]-α-D-glucopyranoside.
m.p.: 51 to 53 °C

Example 66

The corresponding starting compound was treated in the same manner as in Example 12 to give methyl 2,6-dideoxy-2-acetylamino-6-{N-stearoyl-N-[3-(N-phenyl-N-butyrylamino)-propyl]amino}-α-D-glucopyranoside.
IR (neat) $cm^{-1}$: 3600-3100, 1720, 1650
FABMS (m/z): 704 ($MH^+$)

Example 67

The corresponding starting compound was treated in the same manner as in Example 12 to give methyl 2,6-dideoxy-2-acetylamino-6-{N-stearoyl-N-[3-(N-phenyl-N-acetylamino)propyl]amino}-α-D-glucopyranoside.
IR (neat) $cm^{-1}$: 3600-3100, 1650
FABMS (m/z): 676 ($MH^+$)

Example 68

To 1.80 g of methyl 2-deoxy-2-amino-α-D-glucopyranoside dissolved in 20 ml of tetrahydrofuran was added 4.15 g of potassium carbonate dissolved in 20 ml of water, and 3.0 g of stearoyl chloride dissolved in 10 ml of tetrahydrofuran was added dropwise under cooling with ice while stirring. After stirring for 15 minutes, precipitated crystal was filtered, washed with water and dried to give 3.50 g of methyl 2-deoxy-2-stearoylamino-α-D-glucopyranoside.
m.p.: 154 to 155 °C

Example 69

The corresponding starting compound was treated in the same manner as in Example 68 to give methyl 2-deoxy-2-(4-phenylbutyryl)amino-α-D-glucopyranoside.
m.p.: 161 to 163 °C

Example 70

To 3.00 g of methyl 2-deoxy-2-stearoylamino-α-D-glucopyranoside suspended in a tetrahydrofuran/toluene mixture solution (30 ml/30 ml) was added 18 ml of 70 % sodium dihydrobis(2-methoxyethoxy)aluminum solution in toluene, followed by stirring at 60 °C for 2 hours. The organic layer was taken and dried and the solvent was removed by evaporation. The residue was purified by silica gel column chromatography (solvent; chloroform : methanol = 5 : 1) to give 2.68 g of methyl 2-deoxy-2-octadecylamino-α-D-glucopyranoside.
m.p.: 68 to 69 °C

Example 71

The corresponding starting compound was treated in the same manner as in Example 70 to give methyl 2-deoxy-2-(4-phenylbutyl)amino-α-D-glucopyranoside.
IR (neat) $cm^{-1}$: 3600-3100
FABMS (m/z): 326 ($MH^+$)

Examples 72 to 75

The corresponding starting compounds were treated in the same manner as in Example 12 to give compounds shown in Table 10.

Table 10

| Example | 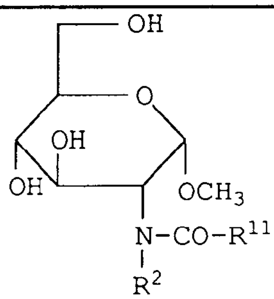 | | |
|---|---|---|---|
| | $R^{11}$ | $R^2$ | Physical properties |
| 72 | $CH_3(CH_2)_{10}-$ | $CH_3(CH_2)_{17}-$ | m.p.: 72-73 °C |
| 73 | $CH{\equiv}C(CH_2)_8-$ | $CH_3(CH_2)_{17}-$ | m.p.: 48-49 °C |
| 74 | $CH_3(CH_2)_{16}-$ | 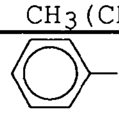 $(CH_2)_4-$ | m.p.: 71-73 °C |
| 75 | $CH_2{=}CH(CH_2)_8-$ | 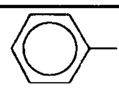 $(CH_2)_4-$ | IR(neat) $cm^{-1}$: 3600-3100, 1620<br>FABMS(m/z): 492 ($MH^+$) |

Example 76

To 18.0 g of methyl 2-deoxy-2-benzyloxycarbonylamino-6-O-tosyl-α-D-glucopyranoside dissolved in 250 ml of toluene was added 19.5 g of 1-amino-4-phenylbutane, followed by heat-reflux overnight. The reaction solution was cooled, an insoluble material was removed by filtration, and toluene was removed by evaporation. The residue was purified by column chromatography [Chromatorex NH-DM 1020 (manufactured by FUJI SILYSIA CHEMICAL Co., Ltd.), solvent: tetrahydrofuran] to give 12.5 g of methyl 2,6-dideoxy-2-benzyloxy-carbonylamino-6-(4-phenylbutyl)amino-α-D-glucopyranoside.
IR (nujol) $cm^{-1}$: 3600-3100, 1720
FABMS (m/z): 459 ($MH^+$)

Examples 77 to 80

The corresponding starting compounds were treated in the same manner as in Example 76 to give compounds shown in Table 11.

Table 11

| Example | 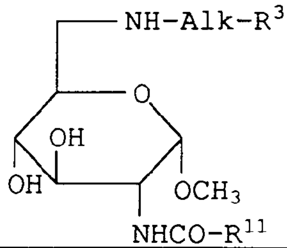 | | |
|---|---|---|---|
| | $R^3$-Alk- | $R^{11}$ | Physical properties |
| 77 | $CH_3(CH_2)_{17}$- | 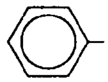 $CH_2$-O- | m.p.: 91-93 °C |
| 78 | 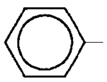 O-$(CH_2)_4$- | 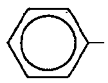 $CH_2$-O- | IR(neat)$cm^{-1}$:3600-3100, 1720<br>FABMS(m/z): 475 ($MH^+$) |
| 79 | 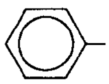 S-$(CH_2)_3$- | 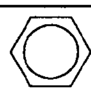 $CH_2$-O- | IR(nujol)$cm^{-1}$:3600-3100, 1690<br>FABMS(m/z): 477 ($MH^+$) |
| 80 | 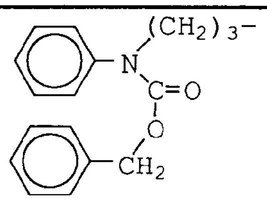 | $(CH_3)_3C$-O- | IR(neat)$cm^{-1}$:3600-3100, 1710<br>FABMS(m/z): 560 ($MH^+$) |

## Examples 81 to 85

The corresponding starting compounds were treated in the same manner as in Example 36 and the obtained compounds were treated in the same manner as in Example 6 or Example 42 to give compounds shown in Table 12.

Table 12

| Example | 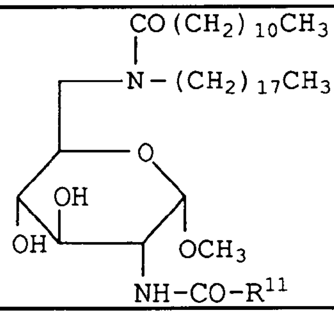 |
| --- | --- |
| | $R^{11}$ |
| 81 | 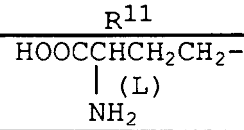 |
| 82 | 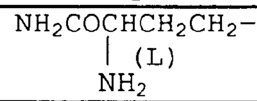 |
| 83 | 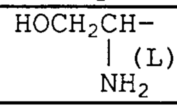 |
| 84 | 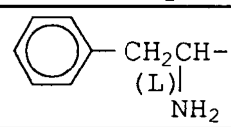 |
| 85 | 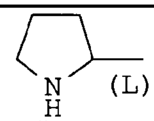 |

Examples 86 to 97

The corresponding starting compounds were treated in the same manners as in Example 1, Example 36 and Example 6 in order to give compounds shown in Table 13 and Table 14.

Table 13

| Example | 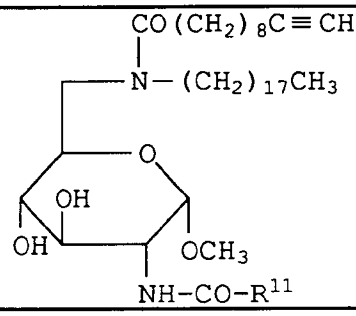 |
|---|---|
| | $R^{11}$ |
| 86 | $HOOCCHCH_2CH_2-$<br>\| (L)<br>$NH_2$ |
| 87 | $NH_2COCHCH_2CH_2-$<br>\| (L)<br>$NH_2$ |
| 88 | $HOCH_2CH-$<br>\| (L)<br>$NH_2$ |
| 89 | $CH_3NHCH_2-$ |
| 90 | 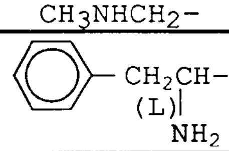 |
| 91 | 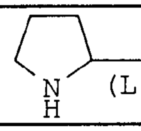 (L) |

Table 14

| Example | 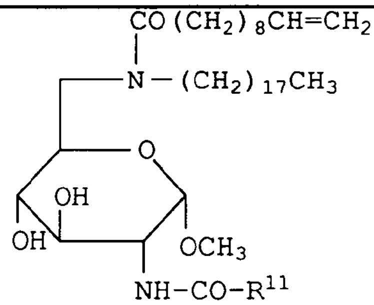 |
|---|---|
| | $R^{11}$ |
| 92 | 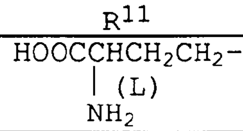 |
| 93 | 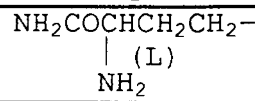 |
| 94 | 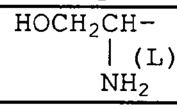 |
| 95 | $CH_3NHCH_2-$ |
| 96 | 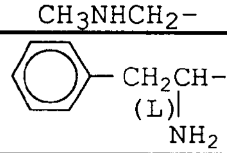 |
| 97 | 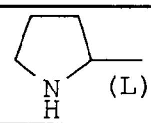 |

Example 98 to 104

The corresponding starting compounds were treated in the same manner as in Example 36 to give compounds shown in Table 15 and Table 16.

Table 15

| Example | 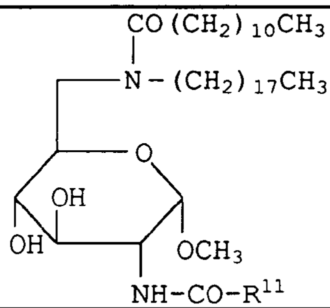 | |
|---|---|---|
| | $R^{11}$ | Physical properties |
| 98 | 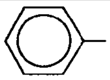 $-CH_2OCONH(CH_2)_2-$ | IR(neat)$cm^{-1}$:3600-3100, 1720, 1650<br>FABMS(m/z): 832($MH^+$) |
| 99 | 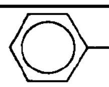 $-CH_2OCONCH_2-$<br>$CH_3$ | IR(neat)$cm^{-1}$:3600-3100, 1710, 1650<br>FABMS(m/z): 832($MH^+$) |
| 100 | 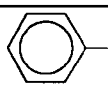 $-CH_2OCONH(CH_2)_3-$ | IR(neat)$cm^{-1}$:3600-3100, 1700, 1630<br>FABMS(m/z): 868 [$(M+Na)^+$] |
| 101 | $NH_2COCH(CH_2)_2-$<br>(D)<br>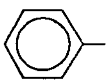 $-CH_2OCONH$ | IR(nujol)$cm^{-1}$:3600-3100, 1670, 1640<br>FABMS(m/z): 889($MH^+$) |
| 102 | 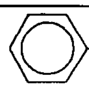 $-NHCH_2-$ | IR(neat)$cm^{-1}$:3600-3100, 1650, 1620<br>FABMS(m/z): 760($MH^+$) |

Table 16

| Example | 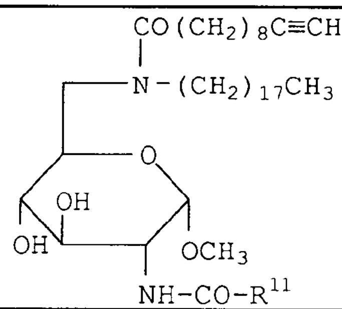 | |
|---|---|---|
| | $R^{11}$ | Physical properties |
| 103 | $(CH_3)_3COCONHCH_2-$ | IR(neat)$cm^{-1}$:3600-3100, 1720, 1650<br>FABMS(m/z): 766($MH^+$) |
| 104 | $(CH_3)_2CHCH_2CH-$<br>(D)<br>$(CH_3)_3COCONH$ | IR(neat)$cm^{-1}$:3600-3100, 1710, 1650<br>FABMS(m/z): 822($MH^+$) |

Examples 105 to 108

The corresponding starting compounds were treated in the same manner as in Example 6 to give compounds shown in Table 17.

Table 17

| Example | 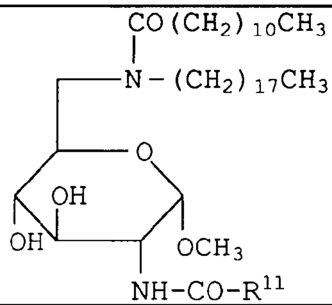 | |
|---|---|---|
| | $R^{11}$ | Physical properties |
| 105 | $NH_2(CH_2)_2-$ | m.p.: 96-97 °C |
| 106 | $CH_3NHCH_2-$ | IR(neat)cm$^{-1}$:3600-3100, 1650<br>FABMS(m/z): 720 [(M+Na$^+$)] |
| 107 | $NH_2(CH_2)_3-$ | IR(nujol)cm$^{-1}$:3600-3100, 1690, 1640<br>FABMS(m/z): 712(MH$^+$) |
| 108 | $NH_2COCH(CH_2)_2-$ (D) with $NH_2$ on CH | IR(neat)cm$^{-1}$:3600-3100, 1640<br>FABMS(m/z): 755(MH$^+$) |

Examples 109 to 110

The corresponding starting compounds were treated in the same manner as in Example 12 to give compounds shown in Table 18.

Table 18

| Example | 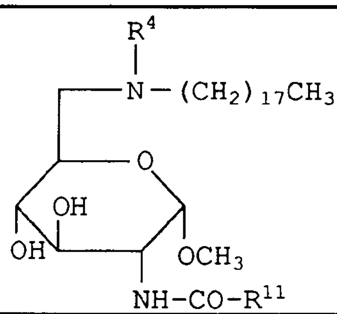 | | |
|---|---|---|---|
| | $R^4$ | $R^{11}$ | Physical properties |
| 109 | $CH{\equiv}C(CH_2)_8CO-$ | $CH_3-$ | IR(neat)cm$^{-1}$:3600-3100, 1640<br>FABMS(m/z): 651(MH$^+$) |
| 110 | $CH_3(CH_2)_{10}CO-$ | $CH_3CH_2OCOCH_2-$ | IR(neat)cm$^{-1}$:3600-3100, 1740, 1650, 1620<br>FABMS(m/z): 741(MH$^+$) |

Examples 111 to 112

The corresponding starting compounds were treated in the same manner as in Example 42 to give compounds shown in Table 19.

Table 19

| Example | 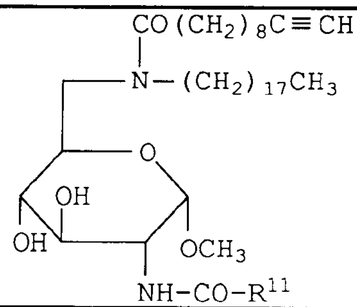 | |
|---|---|---|
| | $R^{11}$ | Physical properties |
| 111 | $NH_2CH_2-$ | IR(neat) $cm^{-1}$: 3600-3100, 1640<br>FABMS (m/z) : 666 ($MH^+$) |
| 112 | $(CH_3)_2CHCH_2CH-$<br>\| (D)<br>$NH_2$ | IR(neat) $cm^{-1}$: 3600-3100, 1650<br>FABMS (m/z) : 722 ($MH^+$) |

Examples 113

To 750 mg of methyl 2,6-dideoxy-2-ethoxycarbonylacetylamino-6-(N-dodecanoyl-N-octadecylamino)-α-D-glucopyranoside dissolved in 10 ml of methanol was added 290 mg of potassium carbonate dissolved in 5 ml of water, followed by stirring at room temperature for 8 hours. To the reaction solution was added 10 % hydrochloric acid aqueous solution to make the solution acidic and the solution was extracted with ethyl acetate. The ethyl acetate layer was washed with water and dried, and the solvent was removed by evaporation. The residue was purified by silica gel column chromatography (solvent; chloroform : methanol = 6 : 1) to give 490 mg of methyl 2,6-dideoxy-2-carboxyacetylamino-6-(N-dodecanoyl-N-octadecylamino)-α-D-glucopyranoside.
IR (neat) $cm^{-1}$: 3600-3100, 1640
FABMS (m/z): 735 [($M+Na^+$)]

Example 114

Methyl 2-deoxy-2-t-butoxycarbonylamino-6-O-tosyl-α-D-glucopyranoside was treated in the same manner as in Example 76 to give methyl 2,6-dideoxy-2-t-butoxycarbonylamino-6-octadecylamino-α-D-glucopyranoside.
IR (neat) $cm^{-1}$: 3600-3100, 1680
FABMS (m/z): 545 ($MH^+$)

Example 115

Methyl 2,6-dideoxy-2-t-butoxycarbonylamino-6-octadecylamino-α-D-glucopyranoside obtained in Example 114 was treated in the same manner as in Example 1 to give methyl 2,6-dideoxy-2-t-butoxycarbonylamino-6-(N-10-undecynoyl-N-octadecylamino)-α-D-glucopyranoside.
IR (neat) $cm^{-1}$: 3600-3100, 1680, 1650
FABMS (m/z): 709 ($MH^+$)

Example 116

Methyl 2,6-dideoxy-2-t-butoxycarbonylamino-6-(N-10-undecynoyl-N-octadecylamino)-α-D-glucopyranoside obtained in Example 116 was treated in the same manner as in Example 10 to give methyl 2,6-dideoxy-2-amino-6-(N-10-undecynoyl-N-octadecylamino)-α-D-glucopyranoside.
IR (neat) $cm^{-1}$: 3600-3100, 1640
FABMS (m/z): 609 ($MH^+$)

Example 117

The corresponding starting compound was treated in the same manner as in Example 36 to give methyl 2,6-dideoxy-2-benzyloxycarbonylaminoacetylamino-6-[N-octadecanoyl-N-(4-phenylbutyl)amino]-α-D-gluco pyranoside.
IR (neat) $cm^{-1}$: 3600-3100, 1720, 1640
FABMS (m/z): 782 ($MH^+$)

Example 118

The corresponding starting compound was treated in the same manner as in Example 6 to give methyl 2,6-dideoxy-2-aminoacetylamino-6-[N-octadecanoyl-N-(4-phenylbutyl)amino]-α-D-glucopyranoside.
IR (neat) $cm^{-1}$: 3600-3100, 1640
FABMS (m/z): 648 ($MH^+$)

Reference example 1

To 5.00 g of methyl 2-deoxy-2-benzyloxycarbonylamino-6-O-tosyl-α-D-glucopyranoside and 2.73 g of di-t-butyl bicarbonate dissolved in 100 ml of methanol was added 1.0 g of 10 % palladium-carbon, followed by catalytic reduction under atmospheric pressure of hydrogen at room temperature. After 5 hours, the catalyst was removed and then methanol was removed by evaporation. The residue was purified by silica gel column chromatography (solvent; chloroform : methanol = 15 : 1) to give 4.65 g of methyl 2-deoxy-2-t-butoxycarbonylamino-6-O-tosyl-α-D-glucopyranoside.
m.p.: 74 to 75 °C

The glucosamine derivative (I) which is the objective compound of the present invention is structurally characterized by an acylamino group introduced at 2,6-positions or 2-position of sugar, and is a useful pharmaceutical compound as a leukocyte-increasing agent, an infection-preventing agent or an anti-infectious activity-facilitating agent since it exhibits an excellent leukocyte-increasing activity, an infection-preventing activity and lower toxicity as compared with a 1,2-substituted sugar which is a known compound.

Concretely, the compound of the present invention, exhibiting a preventing activity or treating activity of leukopenia which is caused by, for example, radiotherapy, a bacterial or fungal infection-preventing activity and/or anti-tumor activity, is useful for general prevention of an injectious disease in a human or an animal, and furthermore can be preferably used in congenital or acquired immunodeficiency, particularly in acquired immunodeficiency caused by severe temporary disorders after radiotherapy or treatment with a material having an immunosuppressive activity. Since the compound of the present invention compensates the immunodeficiency, it can be preferably administered in combination with an anti-infectious or anti-tumor antibiotic or the other chemotherapeutic agent, or in combination with the other medicine.

## Claims

1. A D-glucosamine derivative represented by the formula (I) :

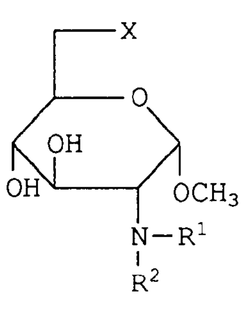

(I)

wherein (1) X is a group represented by the formula:

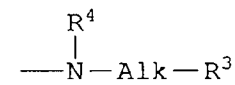

and $R^1$ and $R^2$ are each hydrogen atoms; or
(2) X is hydroxyl group or a group represented by the formula:

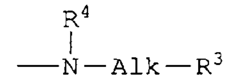

and one of $R^1$ and $R^2$ is a group represented by the formula: $-Y-R^{11}$ or an alkyl group which may have a substituent(s) and the other is hydrogen atom, or one of $R^1$ and $R^2$ is a group represented by the formula: $-Y-R^{11}$ and the other is an alkyl group which may have a substituent(s); Y represents a group represented by the formula: -CO-, -CS- or $-SO_2-$; $R^{11}$ represents an alkyl group which may have a substituent(s), a lower alkoxy group which may have a substituent, an alkenyl group which may have a substituent, an alkynyl group, phenyl group which may have a substituent, a cycloalkyl group, a tricycloalkyl group, a heterocyclic group or an alkylamino group; $R^3$ represents hydrogen atom, phenyl group, phenoxy group, phenylthio group, phenylamino group which may be protected, or a heterocyclic group; $R^4$ represents an alkanoyl group, an alkenoyl group or an alkynoyl group; and Alk represents an alkylene group, provided that a compound in which X is hydroxyl group, one of $R^1$ and $R^2$ is methyl group and the other is acetyl group is excluded,
or a pharmaceutically acceptable salt thereof.

2. The compound as set forth in Claim 1, wherein the alkyl group which may have a substituent(s) is an alkyl group which may be substituted by 1 or 2 groups selected from the group consisting of a lower alkoxycarbonyl group, amino group which may be protected, a carboxyalkanoylamino group, an alkylamino group, phenylamino group, carboxyl group, carbamoyl group which may be substituted by an alkyl group, hydroxyl group which may be protected, a monocyclic or bicyclic heterocyclic group containing sulfur atom or nitrogen atom and phenyl group; the lower alkoxy group which may have a substituent is a lower alkoxy group which may be substituted by phenyl group; the alkenyl group which may have a substituent is an alkenyl group which may be substituted by dimethoxyphenyl group; the phenyl group which may have a substituent is phenyl group which may be substituted by a halogen atom; and the heterocyclic group is a monocyclic or bicyclic heterocyclic group containing sulfur atom or nitrogen atom.

3. The compound as set forth in Claim 2, wherein X is a group represented by the formula:

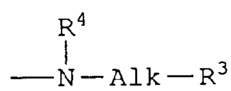

wherein $R^3$ represents hydrogen atom, phenyl group, phenoxy group, phenylthio group, phenylamino group which may be protected, or a heterocyclic group; $R^4$ represents an alkanoyl group, an alkenoyl group or an alkynoyl group; and Alk represents an alkylene group.

4. The compound as set forth in Claim 3, wherein $R^1$ and $R^2$ are each hydrogen atom.

5. The compound as set forth in Claim 4, wherein $R^3$ is hydrogen atom, phenyl group or phenylamino group which may be protected and $R^4$ is an alkanoyl group or an alkynoyl group.

6. The compound as set forth in Claim 3, wherein one of $R^1$ and $R^2$ is a group represented by the formula: $-Y-R^{11}$ and the other is hydrogen atom, and $R^{11}$ represents an alkyl group which may have a substituent(s), a lower alkoxy group which may have a substituent, an alkenyl group which may have a substituent, an alkynyl group, phenyl group which may have a substituent, a cycloalkyl group, a tricycloalkyl group, a heterocyclic group or an alkylamino group.

7. The compound as set forth in Claim 6, wherein $R^3$ is hydrogen atom, phenyl group or phenylamino group which may be protected, and $R^4$ is an alkanoyl group or an alkynoyl group.

**8.** The compound as set forth in Claim 7, wherein Y is a group represented by the formula: -CO-, $R^{11}$ is an alkyl group which may have a substituent(s), and $R^3$ is hydrogen atom or phenyl group.

**9.** The compound as set forth in Claim 8, wherein the alkyl group which may have a substituent(s) is an alkyl group which may be substituted by 1 or 2 groups selected from the group consisting of a lower alkoxycarbonyl group, amino group which may be protected, a carboxyalkanoylamino group, an alkylamino group, phenylamino group, carboxyl group, carbamoyl group, hydroxyl group and phenyl group.

**10.** The compound as set forth in Claim 9, wherein $R^{11}$ is an alkyl group which may be substituted by amino group which may be protected.

**11.** The compound as set forth in Claim 8, wherein the alkyl group which may have a substituent(s) is a group which obtained by removing one carboxyl group from a naturally occurring amino acid or an enantiomer thereof.

**12.** A process for preparing a D-glucosamine derivative represented by the formula (I-a):

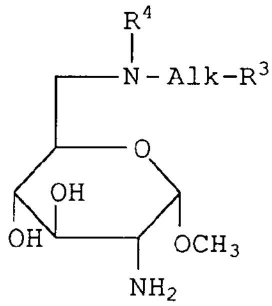

(I-a)

wherein $R^3$ represents hydrogen atom, phenyl group, phenoxy group, phenylthio group, phenylamino group which may be protected, or a heterocyclic group; $R^4$ represents an alkanoyl group, an alkenoyl group or an alkynoyl group; and Alk represents an alkylene group,
or pharmaceutically acceptable salt thereof, which comprises condensing a D-glucosamine compound represented by the formula (II):

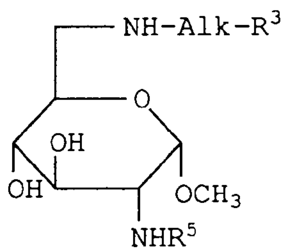

(II)

wherein $NHR^5$ represents amino group which may be protected and the other symbols have the same meanings as above,
or a salt thereof with a compound represented by the formula (III):

$R^4$-OH (III)

wherein the symbols have the same meanings as above, a salt thereof, or a reactive derivative thereof, removing the protective group if required, and converting the resulting compound to a pharmaceutically acceptable salt thereof if required.

**13.** A process for preparing a D-glucosamine derivative represented by the formula (I-b):

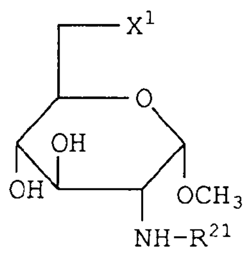

(I-b)

wherein $R^{21}$ represents an alkyl group which may have a substituent(s); and $X^1$ represents hydroxyl group or a group represented by the formula:

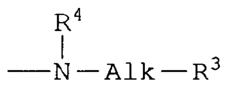

wherein $R^3$ represents hydrogen atom, phenyl group, phenoxy group, phenylthio group, phenylamino group which may be protected, or a heterocyclic group; $R^4$ represents an alkanoyl group, an alkenoyl group or an alkynoyl group; and Alk represents an alkylene group,
or a pharmaceutically acceptable salt, which comprises alkylating a D-glucosamine compound represented by the formula (IV):

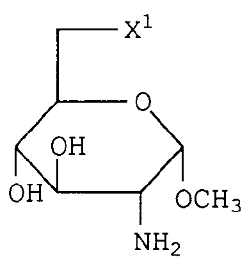

(IV)

wherein the symbols have the same meanings as above, or a salt thereof.

**14.** A process for preparing a D-glucosamine derivative represented by the formula (I-c):

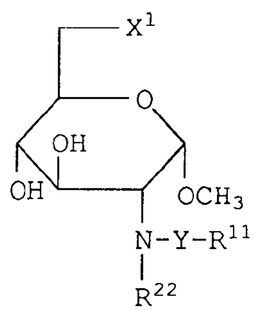

(I-c)

wherein $R^{11}$ represents an alkyl group which may have a substituent(s), a lower alkoxy group which may have a substituent, an alkenyl group which may have a substituent, an alkynyl group, phenyl group which may have a substituent, a cycloalkyl group, a tricycloalkyl group, a heterocyclic group or an alkylamino group; $R^{22}$ represents hydrogen atom or an alkyl group which may have a substituent(s); and $X^1$ represents hydroxyl group or a group represented by the formula:

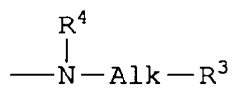

wherein $R^3$ represents hydrogen atom, phenyl group, phenoxy group, phenylthio group, phenylamino group which may be protected, or a heterocyclic group; $R^4$ represents an alkanoyl group, an alkenoyl group or an alkynoyl group; and Alk represents an alkylene group, provided that when $X^1$ is hydroxyl group and $R^{22}$ is methyl group, $R^{11}$-Y- is not acetyl group,
or a pharmaceutically acceptable salt thereof, which comprises condensing a compound represented by the formula (V):

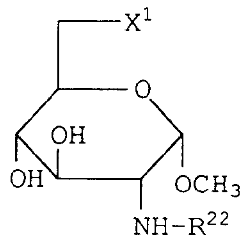

(V)

wherein the symbols have the same meanings as above, or a salt thereof with a compound represented by the formula (VI):

$R^{11}$-Y-OH (VI)

wherein Y represents a group represented by the formula: -CO-, -CS- or $-SO_2-$; and $R^{11}$ has the same meaning as above,
a salt thereof or a reactive derivative thereof.

**15.** A D-glucosamine derivative represented by the formula (II) :

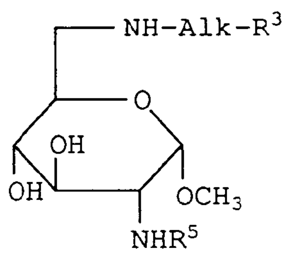

(II)

wherein $R^3$ represents hydrogen atom, phenyl group, phenoxy group, phenylthio group, phenylamino group which may be protected or a heterocyclic group; $NHR^5$ represents amino group which may be protected; and Alk represents an alkylene group.

**16.** A pharmaceutical composition which comprises a therapeutically effective amount of the compound as set forth in Claim 1 in admixture with a conventional pharmaceutically acceptable carrier or diluent.

European Patent Office

# EUROPEAN SEARCH REPORT

Application Number
EP 94 30 8306

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | BULL. CHEM. SOC. JPN., vol.48, 1975 pages 556 - 9 UMEZAWA, S. 'Synthesis of Dihydrostreptobiosamine' * page 557 * | 1,2 | C07H15/12 C07H15/04 C07H15/18 |
| X | US-A-3 767 640 (TETSUO SUAMI) 23 October 1973 * column 3 * | 1,2,14 | |
| X | EP-A-0 062 329 (TANABE SEIYAKU CO.) 13 October 1982 * examples 1-4 * | 1,2 | |
| X | EP-A-0 528 624 (UNILEVER PLC) 24 February 1993 * example 4 * | 1,2 | |
| X | US-A-4 216 208 (PROTER S.P.A.) 5 August 1980 column 6 *compound (i)* | 1,2 | TECHNICAL FIELDS SEARCHED (Int.Cl.6) |
| X | WO-A-93 19761 (IGEN INC.) 14 October 1993 Fig. 13 *compound 31* | 1,2,16 | C07H |
| Y | DATABASE WPI 1990 Derwent Publications Ltd., London, GB; AN 90-071801 'New glucosamine derivatives - useful as immune regulators and antitumor agents' & JP-A-2 025 494 (JAPAN TOBACCO & SALT PUB) 26 January 1990 * abstract * | 1,2,16 | |
| Y | EP-A-0 486 206 (JAPAN TOBACCO INC.) 29 April 1992 * the whole document * | 1,2,16 | |
| | -/-- | | |

The present search report has been drawn up for all claims

| Place of search | Date of completion of the search | Examiner |
|---|---|---|
| MUNICH | 20 January 1995 | Bardili, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 30 8306

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP-A-0 437 016 (SANKYO COMPANY LIMITED) 17 July 1991<br>* the whole document *<br>--- | 1,2,16 | |
| Y | DE-A-39 41 078 (SANDOZ-PATENT-GMBH) 20 June 1991<br>* the whole document *<br>--- | 1,2,16 | |
| Y | US-A-4 323 561 (TEMPLE UNIVERSITY) 6 April 1982<br>* the whole document *<br>--- | 1,2,16 | |
| Y | DE-A-27 08 667 (GIRPI INSTITUTE) 31 August 1978<br>* the whole document *<br>--- | 1,2,16 | |
| A | EP-A-0 149 093 (BAYER AG) 24 July 1985<br>----- | 1-16 | |
| | | | TECHNICAL FIELDS SEARCHED (Int.Cl.6) |

The present search report has been drawn up for all claims

| Place of search | Date of completion of the search | Examiner |
|---|---|---|
| MUNICH | 20 January 1995 | Bardili, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)